# EUROPÄISCHE PATENTANMELDUNG

(11) **EP 3 542 716 A1**
(43) Veröffentlichungstag der Anmeldung: **25.09.2019**
(21) Anmeldenummer: 18163555.8
(22) Anmeldetag: 23.03.2018
(51) Int. Cl.: A61B 5/0424, A61N 1/362, A61N 1/08

(54) **MEDIZINISCHES GERÄT UND METHODE ZUR AUSWERTUNG VON DATEN HINSICHTLICH FEHLER IN EINER ELEKTRODENLEITUNG**

(71) Anmelder: BIOTRONIK SE & Co. KG, 12359 Berlin (DE)
(72) Erfinder: Fischer, René, 10245 Berlin (DE); Busch, Ulrich, 10318 Berlin (DE); Bauditz, Sabrina, 10247 Berlin (DE); Neumann, Andreas, 12437 Berlin (DE); Wohlgemuth, Peter, 09123 Chemnitz (DE)
(74) Vertreter: Keck, Hans-Georg

(57) **Zusammenfassung**

Es wird ein medizinisches Gerät beschrieben, umfassend
• mindestens eine Elektrodenleitung mit mindestens einem Elektrodenpol, die dazu ausgelegt ist, elektrische Potentiale im menschlichen oder tierischen Gewebe zu messen,
• eine Mess- und Steuereinheit, die verbunden ist mit der Elektrodenleitung.

Die Mess- und Steuereinheit ist dazu ausgelegt, Messungen der Impedanz über den Elektrodenpol der Elektrodenleitung zu veranlassen. Die Messungen der Impedanz weist mindestens eine Einzelmessung auf, wobei eine Einzelmessung über ein festgelegtes Zeitfenster stattfindet.

## Beschreibung

Die Erfindung beschreibt ein medizinisches Gerät und eine Methode zur Auswertung von Daten hinsichtlich Fehler in einer Elektrodenleitung oder zwischen Elektrodenleitern oder zwischen Elektrodenleitern und dem Gehäuse.

Medizinische Geräte mit Elektrodenleitern zur Messung von elektrischen Potentialen sowie zur Abgabe von Elektrotherapie sind seit Jahrzehnten bekannt. Derartige Geräte weisen normalerweise ein Gerätegehäuse auf, in dem Energieversorgung, Energiespeicher, Elektronikkomponenten etc. untergebracht sind. Die Elektrodenleitungen sind mit der Elektronik im Gerätegehäuse gekoppelt und führen aus dem Gerätegehäuse heraus.

Ein Beispiel für nicht-implantierbare medizinische Geräte sind externe Elektrokardiogramm (EKG) -Überwachungsgeräte ("Holter-Monitor"), die typischerweise aus einem EKG-Messgerät und mehreren an das Gerät angeschlossenen Elektrodenleitungen bestehen. Am Ende jeder Elektrodenleitung befindet sich eine Messelektrode, die mittels Klebepatch an einem geeigneten Messpunkt auf der Haut des Patienten befestigt werden. Das aufgenommene Messsignal, welches dem Verlauf des elektrischen Potentials von einer Messelektrode zu einer zweiten Messelektrode oder einer Neutralelektrode entspricht, wird im Allgemeinen als "Ableitung" bezeichnet. Ein solcher Holter-Monitor misst und speichert mehrere Ableitungen simultan, die nachträglich dem Arzt zur Analyse zur Verfügung gestellt werden.

TENS (Englisch: Transcutaneous Electrical Nerve Stimulation) - Geräte zur externen transkutanen Elektrostimulation von Nervengewebe werden eingesetzt zur Schmerzbehandlung und Muskelstimulation. Sie sind ein weiteres Beispiel für nicht-implantierbare medizinische Geräte mit Elektrodenleitungen: TENS-Elektrodenleitungen verfügen, ähnlich wie bei Holter-Monitoren, über Patch-Elektroden am distalen Ende. Diese werden im zu stimulierenden Bereich auf der Haut des Patienten aufgeklebt. Über das Gerät werden in der Regel niedrigfrequente Reizstromimpulse abgegeben.

Implantierbare Geräte sind beispielsweise Stimulationsgeräte für das Nervensystem wie Rückenmarkstimulationsgeräte (Engl. "Spinal Cord Stimulator", SCS), Hirnstimulationsgeräte oder Stimulatoren für den Nervus Vagus (Engl. "Vagus Nerve Stimulator", VNS). Ebenfalls seit langem bekannt sind Herzstimulations- und - Herzschrittmachersysteme. Dabei gibt es Herzstimulationssysteme für eine Herzkammer oder mehrere Herzkammern, je nach Bedarf bzw. der Pathophysiologie des Patienten. Auch gibt es Herzstimulationssysteme mit Schockfunktion (implantierbare Kardioverter-Defibrillatorsystemen, Engl. Implantable Cardioverter-Defibrillator, ICD), die in der Lage sind, einen hochenergetischen elektrischen Schock intrakardial zu verabreichen, um lebensgefährliches ventrikuläres Kammerflimmern zu therapieren.

Elektrodenleitungen für implantierbare Systeme weisen normalerweise einen langgestreckten Leitungskörper auf, der aus mehreren elektrischen Leitern besteht, die mit einer elektrischen Isolierung versehen sind. Beispielsweise sind die elektrischen Leiter mit einem Isoliermaterial wie Silikon oder Kunststoff ummantelt. Am proximalen Ende der Elektrodenleitung befinden sich Kontakte, über die eine elektrische Verbindung mit den Komponenten im Gerätegehäuse hergestellt werden kann. Am distalen Ende der Elektrodenleitung und/oder am Leitungskörper befinden sich Elektrodenpole zur Aufnahme eines elektrischen Potentials (z.B. zur Messung einer physiologischen Nervenaktivität) und/oder zur Abgabe von elektrischer Stimulationstherapie. Elektrodenpole können unterschiedlich ausgestaltet sein, beispielsweise als Spitzenelektrode (Englisch "Tip Electrode") oder Ringelektrode; Auch gibt es Elektrodenpole in Form von Schockwendeln diese werden beispielsweise bei ICDs zur Abgabe des intrakardialen Defibrillationsschocks verwendet.

Innerhalb einer Elektrodenleitung sind in der Regel mehrere elektrische Leiter geführt. Die elektrischen Leiter werden zum Beispiel in Form von Seilen oder Wendeln implementiert, um Biegsamkeit und Flexibilität zu gewährleisten.

Durch dauerhafte mechanische Beanspruchung von Elektrodenleitungen können Defekte in der Isolierung sowie in den elektrischen Leitungen auftreten, die zu Leckströmen und Kurzschlüssen sowie zu erhöhten Impedanzen, Wackelkontakten und/ oder Leitungsbrüchen führen können. Ähnlich wie Leitungsbrüche können sich Kontaktierungsprobleme der Abschlussstelle (genannt "Terminal") einer Elektrodenleitung zum Stimulationsgerät äußern. Solche Defekte beeinflussen die Mess-und Elektrotherapiefunktion der Elektrodenleitung ungünstig. In manchen Fällen führt ein Defekt in der Isolierung oder in der elektrischen Leitung zu einem Kurzschluss zwischen zwei Elektrodenleitungen oder zwischen einer Elektrodenleitung und dem Gehäuse des Stimulationsgeräts. In anderen Fällen führt ein solcher Defekt durch Gewebekontakt der defekten Stelle zu Spannungen, die das eigentliche physiologische Messsignal des Patienten überlagern und zu fehlerhaften Detektionen durch das medizinische Gerät führen (z.B. zum soganannten "Oversensing", Detektion von Falsch-Positiven Events). Zeigt sich dies in einer hochfrequenten Störung im Messsignal, kann im Fall eines ICDs eine kardiale Tachykardie durch das Gerät interpretiert werden. Erkennt das Gerät nicht, dass das Signal durch einen Isolierungs- oder Leitungsdefekt verursacht wurde, und nicht durch eine wirkliche patientenseitige Tachykardie, kann eine inadäquate Therapieabgabe die Folge sein. Es kann ein inadäquater Defibrillationsschock abgegeben werden, der äußerst schmerzhaft für den Patienten ist und den es unter allen Umständen zu vermeiden gilt. Leitungsunterbrechungen können zu fatalen Therapieausfällen führen, ebenso besteht grundsätzlich Risiko dass der ICD beschädigt wird, sofern in ein defektes Elektrodensystem Schockenergie abgegeben wird.

Es sind medizinische Geräte bekannt, die mit Elektronik und Algorithmen zur Erkennung von Defekten in der Isolierung oder in elektrischen Leitern einer Elektrodenleitung ausgestattet sind. Beispielsweise wird die elektrische Impedanz zwischen einer Elektrode und dem Gerätegehäuse gemessen. Es sind Geräte bekannt, die über eine solche Impedanzmessung einen einzelnen Impedanzwert einer Ableitung messen. Es werden über einen Zeitraum verteilt (z.B. über einen Tag) einzelne Impedanzwerte generiert, um dann einen Mittelwert aus diesen einzelnen Werten zu berechnen. Bewegt sich dieser Mittelwert innerhalb eines bestimmten Wertebereichs, so gilt die untersuchte Ableitung aus Sicht des Impedanzkriteriums als unauffällig.

US7047083B2 beschreibt eine Methode, bei der in regelmäßigen Abständen ein Impedanzwert für eine Elektrodenleitung aufgenommen wird. Für die aufgenommenen Impedanzwerte wird ein Trend über einen kurzen Zeitraum sowie ein Trend über einen langen Zeitraum bestimmt. Beide Trends werden verglichen, um eine Aussage über den Zustand der Elektrodenleitung zu treffen.

In US8099166B2 wird ein implantierbares kardiales Gerät beschrieben, das ein EKG auf Überschreitungen eines Schwellwerts untersucht. Übersteigt die Anzahl der Überschreitungen einen Grenzwert, wird ein Schnappschuss des EKGs gespeichert.

Typischerweise wird das an einem Schrittmacher oder ICD montierte Elektrodensystem zu einigen wenigen Zeitpunkten am Tag hinsichtlich seiner Impedanz vermessen. Liegen diese Messungen (oftmals noch verrechnet, beispielsweise durch Mittelung) innerhalb eines bestimmten Wertebereiches, so gilt die untersuchte Ableitung aus Sicht des Impedanzkriteriums als unauffällig.

Allerdings weisen die bislang bekannten Systeme den Nachteil auf, dass bestimmte Elektrodendefekte nicht erkannt werden können.

Insbesondere im Fall von Defekten, die nur zwischenzeitlich ein charakteristisches Messsignal verursachen, oder Defekte in der Entstehungsphase, können durch bekannte Detektionsalgorithmen nicht erkannt werden. Solche Defekte werden derzeit nur durch Zufall entdeckt; häufig erst dann, wenn für den Patienten bereits ein Schaden entstanden ist. Insbesondere wenn solche Defekte in Elektrodenleitungen auftreten, über die kein physiologisches Signal gemessen wird, ist eine Detektion über bekannte Algorithmen nicht möglich. Mittels der bekannten Methoden zur Impedanzüberwachung sind solche Defekte ebenso nicht erkennbar. Diese führen Aufnahmen einzelner Impedanzwerte zu definierten Zeiten für eine Ableitung durch, wodurch die Zeitpunkte, in denen der Defekt sich im Impedanzsignal äußert, verpasst werden.

Eine Aufgabe der vorliegenden Erfindung kann darin gesehen werden, eine Methode und eine Vorrichtung zur Detektion bestimmter Szenarien von Defekten einer Elektrode, Elektrodenleitung bzw. Defekte der Isolierung einer Elektrodenleitung bereitzustellen. Ausführungsformen der Erfindung zielen auf die Detektion von sogenannten intermittierenden Defekten ab.

Die vorliegende Erfindung ist gerichtet auf eine verbesserte Detektion von Defekten einer Elektrode, einer Elektrodenleitung bzw. Defekte in der Isolierung einer Elektrodenleitung. Eine Aufgabe der vorliegenden Erfindung kann gesehen werden als Bereitstellung einer Vorrichtung und Methode, um inadäquate Therapien in Folge von fehlerhaft interpretiertem Sensing zu vermeiden

Dabei soll der Stromverbrauch eines medizinischen Gerätes durch die Anwendung der erfindungsgemäßen Methode nur minimal beeinträchtigt werden. Hierfür stellt die Erfindung geeignete Parameter und Parameterbereich bereit, sodass eine hohe Entdeckungswahrscheinlichkeit von besagten Defekten bei geringem Stromverbrauch ermöglicht wird.

Eine weitere Aufgabe der Erfindung kann darin gesehen werden, Leitungsbrüche und lose Headerschrauben zu entdecken und damit die Möglichkeit zur Abgabe effektiver Therapien sicherzustellen.

Als Aufgabe der Erfindung kann die Bereitstellung eines neuartigen Prüfungskriteriums zur Beurteilung von Defekten einer Elektrode, einer Elektrodenleitung oder in der Isolierung verstanden werden.

Die oben beschriebenen Aufgaben werden durch ein medizinisches Gerät gemäß Anspruch 1 gelöst.

Gemäß der vorliegenden Erfindung wird ein medizinisches Gerät beschrieben, umfassend
- mindestens eine Elektrodenleitung mit mindestens einem Elektrodenpol, die dazu ausgelegt ist, elektrische Potentiale im menschlichen oder tierischen Gewebe zu messen,
- eine Mess- und Steuereinheit, die verbunden ist mit der Elektrodenleitung,
wobei die Mess- und Steuereinheit dazu ausgelegt ist, Messungen der Impedanz über den Elektrodenpol der Elektrodenleitung oder über die Elektrodenpole mehrerer Elektrodenleitungen zu veranlassen,
dadurch gekennzeichnet, dass die Messungen der Impedanz zumindest eine Einzelmessung aufweist, und eine Einzelmessung über ein festgelegtes Zeitfenster stattfindet.

Gemäß einer Ausführungsform der Erfindung kann das die Messung der Impedanz kontinuierlich stattfinden, d.h. das Zeitfenster ist nicht in seiner Dauer begrenzt.

Gemäß einer Ausführungsform der Erfindung kann die Messung der Impedanz mehrere Einzelmessungen aufweisen.

Gemäß einem Aspekt der vorliegenden Erfindung weist das Zeitfenster eine Länge von 5 Sekunden bis 360 Sekunden auf. Vorzugsweise, nach einer bevorzugten Ausführungsform der vorliegenden Erfindung, weist das Zeitfenster eine Länge von ungefähr 90 Sekunden auf.

In einer speziellen Ausführungsform der Erfindung weist das Zeitfenster eine Länge von 90 Sekunden auf.

Gemäß einer Ausführungsform der vorliegenden Erfindung handelt es sich bei den Einzelmessungen der Impedanz um Abtastungen mittels einer Samplingfrequenz, im Weiteren bezeichnet als quasi-kontinuierliche Messungen. Alternativ kann es sich bei den Einzelmessungen auch um kontinuierliche Impedanzmessungen handeln.

In einer bevorzugten Ausgestaltung der Erfindung umfasst jede Einzelmessung eine Aufnahme von Impedanzwerten mittels einer Samplingfrequenz, wobei die Samplingfrequenz zwischen 8 Hz und 128 Hz liegt. Nach einer vorteilhaften Ausführungsform der vorliegenden Erfindung umfasst jede Einzelmessung eine Aufnahme von Impedanzwerten mittels einer Samplingfrequenz von ca. 32 Hz.

In einer speziellen Ausführungsform umfasst jede Einzelmessung eine Aufnahme von Impedanzwerten mittels einer Samplingfrequenz von 32 Hz.

Gemäß einer vorteilhaften Realisierung der Erfindung liegt zwischen jeweils zwei Einzelmessungen eine festgelegte Zeitspanne von 0,5 Stunden bis 24 Stunden. In einer bevorzugten Ausführungsform der Erfindung liegt zwischen jeweils zwei Einzelmessungen eine festgelegte Zeitspanne von ungefähr 1 Stunde.

In einem Ausführungsbeispiel werden alle Einzelmessungen einer Gesamtmessung gemeinsam durch die Mess- und Steuereinheit ausgewertet.

Vorzugsweise, nach einem Aspekt der vorliegenden Erfindung, weist eine Gesamtmessung eine festgelegte Anzahl an Einzelmessungen auf. Vorzugsweise findet eine Gesamtmessung über einen festgelegten Zeitraum statt, z.B. 12 Stunden, 24 Stunden, 48 Stunden. Der Zeitraum sollte so gewählt sein, dass eine hinreichende Abdeckung der Patientensicherheit gewährleistet ist, und dass gleichzeitig die Batterielaufzeit durch die Auswertung nicht signifikant reduziert wird.

Vorzugsweise, nach einem Aspekt der vorliegenden Erfindung, ist die Mess-und Steuereinheit dazu ausgelegt, nach Beendung einer Gesamtmessung nahtlos eine nächste Gesamtmessung zu beginnen. Auf diese Weise wird eine kontinuierliche Messabdeckung bereitgestellt.

In einer speziellen Ausführungsform umfasst eine Gesamtmessung einen Zeitraum von 24 Stunden, eine Einzelmessung ein Zeitfenster von 90 Sekunden mit einer Samplingfrequenz von 32 Hz.

Die Auswahl des Zeitraums einer Gesamtmessung, des Zeitfensters für eine Einzelmessung sowie die Samplingfrequenz kann so erfolgen, dass eine hohe Entdeckungswahrscheinlichkeit eines intermittierenden Elektrodenfehlers bei geringem Stromverbrauch des medizinischen Gerätes sichergestellt wird.

Gemäß einer Ausführungsform der Erfindung ist die Mess- und Steuereinheit dazu ausgelegt, die gemessenen Impedanzwerte auszuwerten. Dabei findet die Auswertung hinsichtlich eines Fehlers in der Elektrodenleitung oder in mehreren Elektrodenleitungen oder hinsichtlich Kontaktproblemen einer Steckverbindung statt.
Alternativ kann das medizinische Gerät dazu ausgelegt sein, die gemessenen Impedanzwerte einem externen Gerät oder einem externen Service-Center bereitzustellen, wo sie ausgewertet werden.

Gemäß einer Ausführungsform der Erfindung werden die gemessenen Impedanzwerte ausgewertet, indem die Impedanzwerte oder Differenzwerte zwischen den Impedanzwerten mit einem Schwellwert verglichen werden. Dabei ist der Schwellwert ein vordefinierter Wert, oder kann anhand von aktuellen Messungen der Impedanz regelmäßig aktualisiert werden. Gemäß einer Ausführungsform der Erfindung ist das medizinische Gerät dazu ausgelegt, die gespeicherten Impedanzwerte und/oder die Differenzwerte an ein externes Gerät oder ein externes Service-Center zu versenden.

Gemäß einem Aspekt der Erfindung wertet die Mess- und Steuereinheit gemessene Impedanzwerte aus, indem Differenzwerte zwischen den Impedanzwerten berechnet werden und die Differenzwerte mit einem Schwellwert verglichen werden.

In Ausführungsformen liegt der Schwellwert in einem Bereich von 20 Ohm bis 140 Ohm.

Vorzugsweise, nach einer Ausführungsform der Erfindung, wird ein Differenzwert zwischen zwei diskreten, sukzessiven Impedanzwerten berechnet.

Vorzugsweise, gemäß einem Aspekt der vorliegenden Erfindung ist der Elektrodenpol
- mit einer rechtsventrikulären Schockspule, oder
- mit einer atrialen Schockspule, oder
- mit einer Ring-Elektrode, oder
- mit einer Tip-Elektrode
- der Elektrodenleitung assoziiert.

Gemäß einem Aspekt der Erfindung umfasst das medizinische Gerät ein Gerätegehäuse, wobei das Gerätegehäuse einen elektrischen Pol umfasst bzw. mindestens ein Teil des Gehäuses einen elektrischen Pol bildet. Die Mess-und Steuereinheit ist mit dem Elektrodenpol und dem elektrischen Pol des Gerätegehäuses verbunden. Des Weiteren ist die Mess- und Steuereinheit dazu ausgelegt, Messungen der Impedanz zwischen dem Elektrodenpol und dem elektrischen Pol des Gerätegehäuses durchzuführen.

Nach einer Ausführungsform der Erfindung weist das medizinische Gerät mehr als eine Elektrodenleitung auf. Mindestens eine Elektrodenleitung kann mehr als einen Elektrodenpol aufweisen. Gemäß einem Aspekt der Erfindung ist die Mess- und Steuereinheit dazu ausgelegt, Messungen der Impedanz jeweils zwischen zwei Polen durchzuführen, wobei die zwei Pole mindestens eine Kombination aus den Elektrodenpolen, und/oder dem elektrischen Pol des Gerätegehäuses umfassen.

Nach einer Ausgestaltung des erfindungsgemäßen medizinischen Geräts werden durch die Mess- und Steuereinheit jeweils für eine Kombination aus den Elektrodenpolen, und/oder dem elektrischen Pol des Gerätegehäuses einzeln Impedanzwerte ausgewertet.

Gemäß einer bevorzugten Ausführungsform der Erfindung ist die Mess- und Steuereinheit dazu ausgelegt, für eine Einzelmessung bei Überschreitung eines Schwellwertes durch den berechneten Differenzwert einen Zählerwert zu inkrementieren, und bei Überschreitung eines Zählerlimits eine Speicherung der gemessenen Impedanzwerte auszulösen.

Vorzugsweise, nach einer Ausgestaltung der vorliegenden Erfindung, ist die Mess- und Steuereinheit ausgelegt, eine Speicherung von Impedanzwerten einer Einzelmessung aus einer Reihe von jener Einzelmessungen zu veranlassen, die aus der Reihe von Einzelmessungen die Einzelmessung mit dem höchsten Zählerwert darstellt. Alternativ werden die Impedanzwerte einer Einzelmessung gespeichert, die die chronologisch erste Einzelmessung aus der Reihe von Einzelmessungen darstellt, für die das Zählerlimit überschritten wurde.. Gemäß einer Ausführungsform werden bei der Speicherung die Impedanzwerte innerhalb eines Unterzeitfensters gespeichert. Vorzugsweise ist das Unterzeitfenster mit dem Zeitpunkt der Überschreitung des Zählerlimits verbunden.

Nach einem Aspekt der vorliegenden Erfindung ist das medizinische Gerät dazu ausgelegt, die gespeicherten Impedanzwerte an ein externes Gerät oder ein externes Service-Center zu versenden.

Nach einem Aspekt der vorliegenden Erfindung ist das medizinische Gerät ausgeführt als implantierbares Stimulationsgerät für die Neurostimulation, wie z.B. für die Rückenmarkstimulation oder Vagus Nerv Stimulation;
kardiales Stimulationsgerät, wie z.B. ein Herzschrittmacher, ein ICD oder ein CRT-Gerät.

Gemäß einer Ausführungsform der vorliegenden Erfindung, bei der das medizinische Gerät als ICD ausgeführt ist, ist die Mess- und Steuereinheit dazu ausgelegt, Messungen der Impedanz jeweils zwischen zwei Polen durchzuführen, wobei die zwei Pole mindestens eine Kombination aus folgenden umfassen:
- Rechtsventrikuläre Wendelelektrode und Gerätegehäuse,
- Rechtsventrikuläre Wendelelektrode und rechtsventrikuläre Tip-Elektrode,
- Rechtsventrikuläre Ring-Elektrode und Gerätegehäuse,
- Rechtsventrikuläre Ring-Elektrode und rechtsventrikuläre Tip-Elektrode,
- SVC Wendelelektrode und Gerätegehäuse, und/oder
- SVC Wendelelektrode und rechtsventrikuläre Wendelelektrode.

Zudem wird eine Methode zur Auswertung von Impedanzwerten hinsichtlich eines Fehlers in einer Elektrodenleitung beschrieben. Die Methode umfasst die Schritte:
- Messungen einer Impedanz über den Elektrodenpol einer Elektrodenleitung,
- Auswertung gemessener Impedanzwerte hinsichtlich eines Fehlers in der Elektrodenleitung,
- dadurch gekennzeichnet, dass die Messungen der Impedanz zumindest eine Einzelmessung aufweist, und eine Einzelmessung über ein festgelegtes Zeitfenster stattfindet.

Gemäß einer Ausführungsform des erfindungsgemäßen Verfahrens kann das die Messung der Impedanz kontinuierlich stattfinden, d.h. das Zeitfenster ist nicht in seiner Dauer begrenzt.

Gemäß einer Ausführungsform des erfindungsgemäßen Verfahrens kann die Messung der Impedanz mehrere Einzelmessungen aufweisen.

Verschiedene Ausführungsformen und Aspekte der erfindungsgemäßen Vorrichtung sind ebenfalls im Rahmen der Erfindung anwendbar auf die korrespondierende Methode zur Auswertung von Impedanzwerten hinsichtlich eines Fehlers in einer Elektrodenleitung.

Gemäß einer Ausführungsform der vorliegenden Erfindung sollen mindestens über einen Zeitraum hinweg pro Tag Impedanzmessungen auf den relevanten Messableitungen vorgenommen werden. Bei jeder Einzelmessung wird die Impedanz über ein vorbestimmtes Zeitfenster gemessen, wobei die Samples des abgetasteten Impedanzsignals gespeichert werden. Gemäß einem Aspekt der Erfindung werden diese Messungen im medizinischen Gerät und/oder in einem externen Gerät ausgewertet. Fällt bei der Auswertung eine Besonderheit auf, die nicht dem erwarteten, physiologisch typischen Impedanzverlauf entspricht, wird ein Zähler inkrementiert. Ist das medizinische Gerät ein kardiales Implantat wie z.B. ein ICD, handelt es sich bei dem physiologisch typischen Impedanzverlauf um solche, die durch die Herzbewegung hervorgerufen werden.

Gemäß einer Ausführungsform der vorliegenden Erfindung wird der Zählerwert des inkrementierten Zählers nach einer vorbestimmten Zeit ausgewertet. So kann der Zustand eines Defekts in der Elektrodenleitung und/oder in der Isolierung erkannt werden, wenn der Zählerwert ein Zählerlimit überschreitet.

Nach einem Aspekt der vorliegenden Erfindung wird nach Erkennung eines Defekts eine entsprechende Folgemaßnahme durch das medizinische Gerät und/oder das externe Gerät eingeleitet. Beispielsweise kann durch das medizinische Gerät ein Alarm an ein externes Gerät und/oder ein externes Datencenter abgesetzt werden. Verfügt das medizinische Gerät über mehrere Elektrodenpole, für die individuell eine Detektion von Defekten gemäß der Erfindung durchgeführt wird, und wird ein Defekt in Zusammenhang mit einer Elektrode (oder Ableitung) entdeckt, kann das Gerät so konfiguriert sein, dass eine automatische Umschaltung des Strompfades vorgenommen wird. Beispielsweise kann auf eine Elektrode geschaltet werden, die räumlich am nächsten angeordnet ist zu jener, bei der der Defekt erkannt wurde. Alternativ kann die betroffene Elektrode temporär deaktiviert werden.

Ist das medizinische Gerät beispielsweise ein ICD und wird ein Defekt im Zusammenhang mit einem Elektrodenpol mit Sensingfunktion erkannt, kann das Gerät automatisch auf eine andere Ableitung für die Messung schalten. Wird ein Defekt im Zusammenhang mit einer Schockspule bzw. Schockwendel erkannt, so kann das Gerät automatisch den Schockpfad ändern. Auf diese Weise kann verhindert werden, dass durch einen sich gerade ausbildenden Defekt in der Elektrodenleitung (bzw. an der Elektrode oder in der Isolierung der Elektrode) es zur Abgabe inadäquater Therapien kommen kann (bedingt durch fehlerhaftes Sensing) oder dass es zum Ausbleiben eines notwendigen Schocks kommt (bedingt durch einen Defekt in der Elektrodenleitung mit Schockfunktion).

Die vorliegende Erfindung bietet mehrere Vorteile. Zum einen wird eine Überwachung der Funktionalität von Elektrodenleitungen bezüglich intermittierend auftretender Defekte ohne aktive Beteiligung des Arztes auf zuverlässige Weise möglich. Im Zusammenhang mit der Auslösung eines automatisierten Hinweises / Alarms bei Erkennung eines Defekts z.B. an ein externes Gerät oder ein externes Datencenter ist ein Prozess für eine zuverlässige frühzeitige Benachrichtigung solcher Defekte implementiert werden.

Auch bietet die vorliegende Erfindung eine hohe Sensibilität und Spezifität (d.h. Vermeidung falsch-positiver Alarme) für die Erkennung besagter Defekt-Szenarien. Die erfindungsgemäße Vorrichtung und Methode ermöglicht eine zuverlässige Unterscheidung zwischen durch besagte Defekte erzeugte Impedanzsignalverläufe und jene, die durch physiologische Ereignisse des Patienten erzeugte werden, wie z.B. durch körperliche Bewegung.

Gemäß einer Ausführungsform der vorliegenden Erfindung sollen mindestens über einen Zeitraum hinweg pro Tag Impedanzmessungen auf den relevanten Messableitungen vorgenommen werden. Bei jeder Einzelmessung wird die Impedanz über ein vorbestimmtes Zeitfenster gemessen, wobei das Impedanzsignal mittels einer Abtastfrequenz Sample-Weise gespeichert wird. Gemäß einem Aspekt der Erfindung werden diese Messungen im medizinischen Gerät und/oder in einem externen Gerät ausgewertet. Fällt bei der Auswertung eine Besonderheit auf, die nicht dem erwarteten, physiologisch typischen Impedanzverlauf entspricht, wird ein Zähler inkrementiert. Ist das medizinische Gerät ein kardiales Implantat wie z.B. ein ICD, handelt es sich bei dem physiologisch typischen Impedanzverlauf um solche, die durch die Herzbewegung hervorgerufen werden.

Gemäß einer Ausführungsform der vorliegenden Erfindung wird der Zählerwert des inkrementierten Zählers nach einer vorbestimmten Zeit ausgewertet. So kann der Zustand eines Defekts in der Elektrodenleitung und/oder in der Isolierung erkannt werden, wenn der Zählerwert ein Zählerlimit überschreitet.

Nach einem Aspekt der vorliegenden Erfindung wird nach Erkennung eines Defekts eine entsprechende Folgemaßnahme durch das medizinische Gerät und/oder das externe Gerät eingeleitet. Beispielsweise kann durch das medizinische Gerät ein Alarm an ein externes Gerät und/oder ein externes Datencenter abgesetzt werden. Verfügt das medizinische Gerät über mehrere Elektrodenpole, für die individuell eine Detektion von Defekten gemäß der Erfindung durchgeführt wird, und wird ein Defekt in Zusammenhang mit einer Elektrode (oder Ableitung) entdeckt, kann das Gerät so konfiguriert sein, dass eine automatische Umschaltung des Strompfades vorgenommen wird. Beispielsweise kann auf eine Elektrode geschaltet werden, die räumlich am nächsten angeordnet ist zu jener, bei der der Defekt erkannt wurde. Alternativ kann die betroffene Elektrode temporär deaktiviert werden.

Ist das medizinische Gerät beispielsweise ein ICD und wird ein Defekt im Zusammenhang mit einem Elektrodenpol mit Sensingfunktion erkannt, kann das Gerät automatisch auf eine andere Ableitung für die Messung schalten. Wird ein Defekt im Zusammenhang mit einer Schockspule bzw. Schockwendel erkannt, so kann das Gerät automatisch den Schockpfad ändern. Auf diese Weise kann verhindert werden, dass durch einen sich gerade ausbildenden Defekt in der Elektrodenleitung (bzw. an der Elektrode oder in der Isolierung der Elektrode) es zur Abgabe inadäquater Therapien kommen kann (bedingt durch fehlerhaftes Sensing) oder dass es zum Ausbleiben eines notwendigen Schocks kommt (bedingt durch einen Defekt in der Elektrodenleitung mit Schockfunktion).

Die vorliegende Erfindung bietet mehrere Vorteile. Zum einen wird eine Überwachung der Funktionalität von Elektrodenleitungen bezüglich intermittierend auftretender Defekte ohne aktive Beteiligung des Arztes auf zuverlässige Weise möglich. Im Zusammenhang mit der Auslösung eines automatisierten Hinweises / Alarms bei Erkennung eines Defekts z.B. an ein externes Gerät oder ein externes Datencenter ist ein Prozess für eine zuverlässige frühzeitige Benachrichtigung solcher Defekte implementiert werden.

Auch bietet die vorliegende Erfindung eine hohe Sensibilität und Spezifität für die Erkennung besagter Defekt-Szenarien. Die erfindungsgemäße Vorrichtung und Methode ermöglicht eine zuverlässige Unterscheidung zwischen durch besagte Defekte erzeugte Impedanzsignalverläufe und jene, die durch physiologische Ereignisse des Patienten erzeugte werden, wie z.B. durch körperliche Bewegung.

Die Erfindung ist nachfolgend beispielhaft, anhand von einem in Zeichnungen dargestellten Ausführungsbeispiel, näher erläutert. Es zeigen in schematischer Darstellung:
- Fig. 1: Ausführungsbeispiel für die vorliegende Erfindung anhand eines Stimulationsgeräts mit einer Ring-Elektrode und einer Tip-Elektrode
- Fig. 2: Ausführungsbeispiel für die vorliegende Erfindung anhand eines Stimulationsgeräts mit einer Ring-Elektrode, einer Tip-Elektrode und einer Schockwendel
- Fig. 3: Ausführungsbeispiel für die vorliegende Erfindung anhand eines Stimulationsgeräts mit einer Ring-Elektrode, einer Tip-Elektrode und zwei Schockwendeln

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

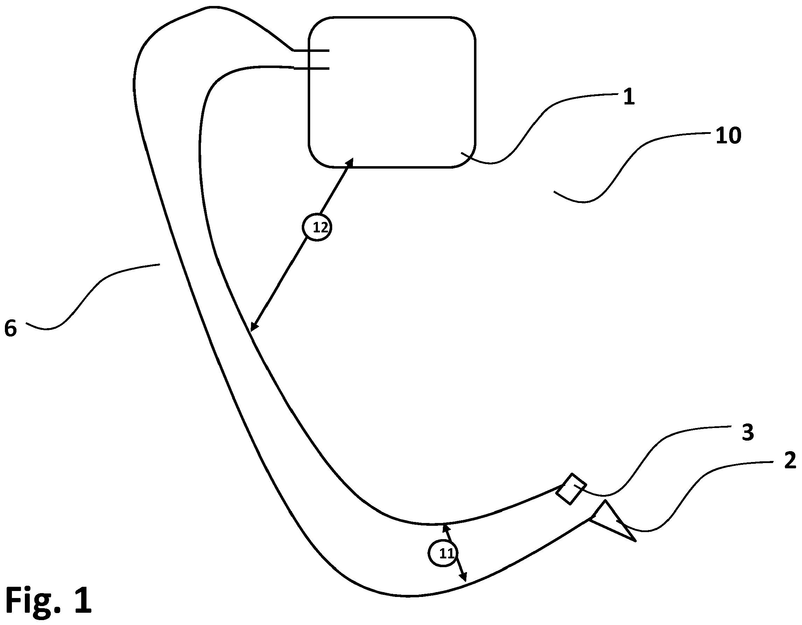

Figur 1 zeigt ein Ausführungsbeispiel für die vorliegende Erfindung anhand eines Stimulationsgeräts 10 mit einer Ring-Elektrode und einer Tip-Elektrode. Konkrete Ausgestaltungen sind implantierbare Herzschrittmacher, Hirnschrittmacher, Rückenmarkstimulatoren oder dergleichen. Das Stimulationsgerät 10 umfasst Gerätegehäuse 1, Tip-Elektrode 2, Ring-Elektrode 3 sowie elektrische Zuleitungen 6 für die Elektroden. Gemäß der Erfindung kann die Messung der Impedanz zur Detektion von Defekten der Elektrode, der Elektrodenleiter oder der Isolierung beispielsweise über
- Ableitung 11 (zwischen Tip-Elektrode 2 und Ring-Elektrode 3) und/ oder
- Ableitung 12 (zwischen Ring-Elektrode 3 und Gerätegehäuse 1)
durchgeführt werden.

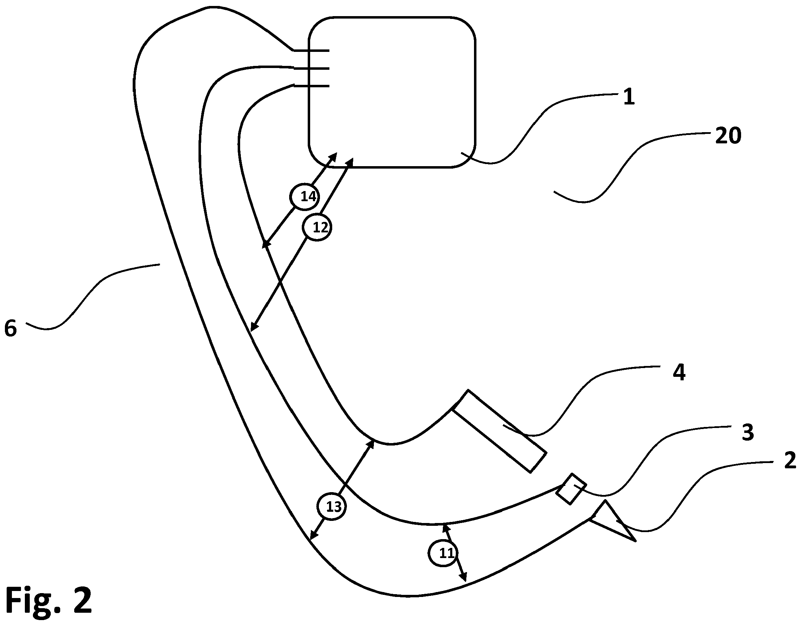

Figur 2 zeigt ein Ausführungsbeispiel für die vorliegende Erfindung anhand eines Stimulationsgeräts 20 mit einer Ring-Elektrode, einer Tip-Elektrode und einer Schockwendel 4. Eine konkrete Ausgestaltung ist ein implantierbarer Herzschrittmacher-Kardioverter mit Defibrillationsfunktion (ICD). Das Stimulationsgerät 20 umfasst Gerätegehäuse 1, Tip-Elektrode 2, Ring-Elektrode 3, Schockwendel 4 sowie elektrische Zuleitungen 6 für die Elektroden. Gemäß der Erfindung kann die Messung der Impedanz zur Detektion von Defekten der Elektrode, der Elektrodenleitung oder der Isolierung beispielsweise über
Ableitung 11 (zwischen Tip-Elektrode 2 und Ring-Elektrode 3) und/oder
Ableitung 12 (zwischen Ring-Elektrode 3 und Gerätegehäuse 1) und/oder
Ableitung 13 (zwischen Tip-Elektrode und Schockwendel 4) und/oder
Ableitung 14 (zwischen Schockwendel 4 und Gerätegehäuse 1)
durchgeführt werden.

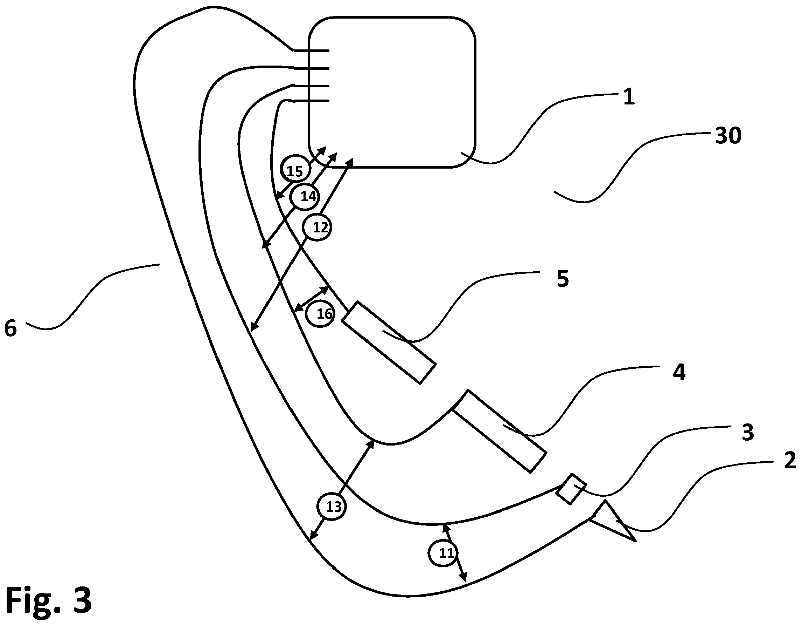

Figur 3 zeigt ein Ausführungsbeispiel für die vorliegende Erfindung anhand eines Stimulationsgeräts 30 mit einer Ring-Elektrode, einer Tip-Elektrode, einer Schockwendel 4 und einer weiteren Schockwendel 5. Eine konkrete Ausgestaltung ist ein implantierbarer Herzschrittmacher-Kardioverter mit Defibrillationsfunktion (ICD) mit einer rechtsventrikulären Schockwendel und einer SVC Schockwendel. Das Stimulationsgerät 30 umfasst Gerätegehäuse 1, Tip-Elektrode 2, Ring-Elektrode 3, Schockwendel 4, Schockwendel 5 sowie elektrische Zuleitungen 6 für die Elektroden. Gemäß der Erfindung kann die Messung der Impedanz zur Detektion von Defekten der Elektrode, der Elektrodenleitung oder der Isolierung beispielsweise über
Ableitung 11 (zwischen Tip-Elektrode 2 und Ring-Elektrode 3) und/oder
Ableitung 12 (zwischen Ring-Elektrode 3 und Gerätegehäuse 1) und/oder
Ableitung 13 (zwischen Tip-Elektrode und Schockwendel 4) und/oder
Ableitung 14 (zwischen Schockwendel 4 und Gerätegehäuse 1) und/oder
Ableitung 15 (zwischen Schockwendel 5 und Gerätegehäuse 1) und/oder
Ableitung 16 (zwischen Schockwendel 4 und Schockwendel 5)
durchgeführt werden.

Gemäß einem Aspekt der Erfindung werden Impedanzänderungen oberhalb einer bestimmten Slewrate detektiert und ausgewertet. Vorzugsweise, nach einer Ausführungsform der Erfindung, wird für die Signalauswertung ein Differenzwert zwischen zwei diskreten, sukzessiven Impedanzwerten berechnet. Es werden gemäß eines Beispiels Messungen der Impedanz jeweils zwischen zwei elektrischen Polen durchgeführt, wobei die zwei Pole mindestens eine Kombination aus folgenden umfassen:
- Rechtsventrikuläre Wendelelektrode und Gerätegehäuse,
- Rechtsventrikuläre Wendelelektrode und rechtsventrikuläre Tip-Elektrode,
- Rechtsventrikuläre Ring-Elektrode und Gerätegehäuse,
- Rechtsventrikuläre Ring-Elektrode und rechtsventrikuläre Tip-Elektrode,
- SVC Wendelelektrode und Gerätegehäuse, und/oder
- SVC Wendelelektrode und rechtsventrikuläre Wendelelektrode.

Gemäß einer bevorzugten Ausführungsform der Erfindung ist die Mess- und Steuereinheit dazu ausgelegt, für eine Einzelmessung bei Überschreitung eines Schwellwertes durch den berechneten Differenzwert einen Zählerwert zu inkrementieren, und bei Überschreitung eines Zählerlimits eine Speicherung der gemessenen Impedanzwerte auszulösen.
Der besagte Schwellwert ist dabei, gemäß einem Aspekt der Erfindung, abhängig von der betrachteten Ableitung.

Gemäß einer Ausführungsform, bei der das medizinische Gerät vorzugsweise ausgeführt ist als implantierbares kardiales Therapiegerät, kann zumindest ein Zähler für die jeweilige Ableitung inkrementiert werden, wenn die Impedanzdifferenz sampleweise höher gemessen wird als:
- Rechtsventrikuläre Wendelelektrode und Gerätegehäuse : ca. oder genau 24 Ohm,
- Rechtsventrikuläre Wendelelektrode und rechtsventrikuläre Tip-Elektrode: ca. oder genau 78 Ohm,
- Rechtsventrikuläre Ring-Elektrode und Gerätegehäuse: ca. oder genau 78 Ohm,
- Rechtsventrikuläre Ring-Elektrode und rechtsventrikuläre Tip-Elektrode: ca. oder genau 137 Ohm,
- SVC Wendelelektrode und Gerätegehäuse: ca. oder genau 24 Ohm, und/oder
- SVC Wendelelektrode und rechtsventrikuläre Wendelelektrode: ca. oder genau 40 Ohm.

Gemäß einer Ausführungsform wird die Häufigkeit dieser Ereignisse pro Zeitabschnitt (z.B. tagesweise) gezählt und ausgewertet.

Gemäß einer Ausführungsform alternativ oder ergänzend zur oben beschriebenen Ausführungsform kann die absolute Impedanz sampleweise gegen einen absoluten Schwellwert verglichen werden. Der absolute Schwellwert kann dabei spezifisch für die verwendete Elektrode parametriert sein oder automatisch aus den vorherigen Messungen (z.B. aus Trenddaten) laufend nachgeführt werden.

### Bezugszeichen

- 1: Gerätegehäuse
- 2: Tip-Elektrode
- 3: Ring-Elektrode
- 4: Schockwendel
- 5: Schockwendel
- 6: Elektrodenleitungen
- 10: Medizinisches Gerät
- 11: Ableitung Tip-Elektrode 2 zu Ring-Elektrode 3
- 12: Ableitung Ring-Elektrode 3 zu Gerätegehäuse 1
- 13: Ableitung Tip-Elektrode zu Schockwendel 4
- 14: Ableitung Schockwendel 4 zu Gerätegehäuse 1
- 15: Ableitung Schockwendel 5 zu Gerätegehäuse 1
- 16: Ableitung Schockwendel 4 zu Schockwendel 5
- 20: Medizinisches Gerät
- 30: Medizinisches Gerät

**Begriffsdefinitionen und Abkürzungen**

| | |
|---|---|
| Ableitung / Messableitung | Unter Ableitung oder Messableitung wird im Sinne der Erfindung ein aufgenommenes Messignal verstanden, welches dem Verlauf des elektrischen Potentials von einer Messelektrode zu einer zweiten Messelektrode oder einer Neutralelektrode entspricht. |
| Distales Ende | Für eine Elektrodenleitung das Ende, das fern vom Gerätgehäuse angeordnet ist. |
| EKG | Elektrokardiogramm |
| Elektrode / Elektrodenpol | Unter dem Begriff Elektrode oder Elektrodenpol wird im Zusammenhang der Erfindung eine mit einem Elektrodenleiter verbundene metallische Einheit verstanden, die in Kombination mit einer zweiten Elektrode bzw. einer Gegenelektrode eine Aufnahme von Spannungspotentialen oder eine Stromabgabe ermöglicht. |
| Elektrodenleitung | Eine Elektrodenleitung wird im Sinne der Erfindung verstanden als Zuleitung aus elektrisch leitendem Material. Am Ende und/oder entlang der Elektrodenleitung können sich Elektroden bzw. Elektrodenpole zur Messung von Spannungspotentialen oder zur Stromabgabe befinden. Eine Elektrodenleitung wird in der Regel gebildet aus mehreren gegeneinander isolierten Elektrodenleitern (auch als Adern bezeichnet). |
| ICD | Englisch: Implantable Cardioverter-Defibrillator |
| IEGM | Intrakardiales Elektrogram (Englisch: intracardial electrogram) |
| Intermittierend | Als 'intermittierend' wird im Kontext der Erfindung ein Ereignis beschrieben, das zeitweise und nicht-kontinuierlich auftritt. |
| Isolierung | Unter der Isolierung wird das Mittel zur elektrischen Isolierung einer Elektrodenleitung verstanden. |
| Proximales Ende | Für eine Elektrodenleitung das Ende, das an das Gerätegehäuse gekoppelt ist. |
| Quasi-kontinuierlich | Als 'quasi-kontinuierlich' wird im Sinne der Erfindung eine Messung beschrieben, die über einen gewissen Zeitraum oder über ein gewisses Zeitfenster durchgehend stattfindet. Im Fall von Messungen von Impedanzwerten, die über eine Abtastfrequenz sampleweise erfasst werden, handelt es sich rein technisch gesehen nicht um eine 'kontinuierliche' Messung (wie es bei einem analogen Messsignal der Fall wäre) daher der Begriff "quasikontinuierlich'. Die Erfindung soll jedoch nicht beschränkt sein auf eine quasi-kontinuierliche Messung der Impedanz. |
| SCS | Englisch: Spinal Cord Stimulation / Spinal Cord Stimulator |
| Sensing | Unter dem Begriff Sensing wird im Kontext der Erfindung die Erfassung von physiologischen Signalen über die Messung von elektrischen Potentialen verstanden. |
| SVC | Superior Vena Cava |
| TENS | Englisch: Transcutaneous Electrical Nerve Stimulation |
| VNS | Englisch: Vagus Nerve Stimulation |

## Patentansprüche

1. Medizinisches Gerät, umfassend
• mindestens eine Elektrodenleitung mit mindestens einem Elektrodenpol, die dazu ausgelegt ist, elektrische Potentiale im menschlichen oder tierischen Gewebe zu messen,
• Mess- und Steuereinheit, die verbunden ist mit der Elektrodenleitung,
wobei die Mess- und Steuereinheit dazu ausgelegt ist, Messungen der Impedanz über den Elektrodenpol der Elektrodenleitung zu veranlassen,
**dadurch gekennzeichnet, dass** die Messungen der Impedanz mehrere Einzelmessungen aufweist, und eine Einzelmessung über ein festgelegtes Zeitfenster stattfindet.

2. Medizinisches Gerät nach Anspruch 1, **dadurch gekennzeichnet, dass** das Zeitfenster eine Länge von 5 Sekunden bis 360 Sekunden aufweist.

3. Medizinisches Gerät nach einem der vorhergehenden Ansprüche, **dadurch gekennzeichnet, dass** jede Einzelmessung eine Aufnahme von Impedanzwerten mittels einer Samplingfrequenz umfasst, wobei die Samplingfrequenz zwischen 8 Hz und 128 Hz liegt.

4. Medizinisches Gerät nach mindestens einem der vorhergehenden Ansprüche, wobei zwischen jeweils zwei Einzelmessungen eine festgelegte Zeitspanne von 0,5 Stunden bis 24 Stunden liegt.

5. Medizinisches Gerät nach mindestens einem der vorhergehenden Ansprüche, wobei zwischen jeweils zwei Einzelmessungen eine festgelegte Zeitspanne von ungefähr 1 Stunde liegt.

6. Medizinisches Gerät nach mindestens einem der vorhergehenden Ansprüche, **dadurch gekennzeichnet, dass** die Mess- und Steuereinheit dazu ausgelegt ist, die gemessenen Impedanzwerte auszuwerten oder das medizinische Gerät dazu ausgelegt ist, die gemessenen Impedanzwerte einem externen Gerät oder einem externen Service-Center bereitzustellen, wo sie ausgewertet werden,

7. Medizinisches Gerät nach Anspruch 6, wobei die gemessenen Impedanzwerte ausgewertet werden, indem die Impedanzwerte oder Differenzwerte zwischen den Impedanzwerten mit einem Schwellwert verglichen werden, wobei der Schwellwert ein vordefinierter Wert ist, oder wobei der Schwellwert einen Wert dargestellt, der anhand von aktuellen Messungen der Impedanz regelmäßig aktualisiert wird, wobei das medizinische Gerät dazu ausgelegt ist, die gespeicherten Impedanzwerte und/oder die Differenzwerte an ein externes Gerät oder ein externes Service-Center zu versenden.

8. Medizinisches Gerät nach Anspruch 6 oder 7, **dadurch gekennzeichnet, dass** ein Differenzwert zwischen zwei diskreten, sukzessiven Impedanzwerten berechnet wird.

9. Medizinisches Gerät nach mindestens einem der vorhergehenden Ansprüche,
**dadurch gekennzeichnet, dass** der Elektrodenpol
mit einer rechtsventrikulären Schockspule, oder
mit einer atrialen Schockspule, oder
mit einer Ring-Elektrode, oder
mit einer Tip-Elektrode
der Elektrodenleitung assoziiert ist, und wobei
das medizinische Gerät ein Gerätegehäuse umfasst, und das Gerätegehäuse einen elektrischen Pol umfasst,
wobei die Mess-und Steuereinheit mit dem Elektrodenpol und dem elektrischen Pol des Gerätegehäuses verbunden ist,
wobei die Mess- und Steuereinheit dazu ausgelegt ist, Messungen der Impedanz zwischen dem Elektrodenpol und dem elektrischen Pol des Gerätegehäuses durchzuführen.

10. Medizinisches Gerät nach Anspruch 9, wobei das medizinische Gerät mehr als eine Elektrodenleitung aufweist, und/oder die Elektrodenleitung mehr als einen Elektrodenpol aufweist, und
die Mess- und Steuereinheit dazu ausgelegt ist, Messungen der Impedanz jeweils zwischen zwei Polen durchzuführen, wobei die zwei Pole mindestens eine Kombination aus den Elektrodenpolen, und/oder dem elektrischen Pol des Gerätegehäuses umfassen.

11. Medizinisches Gerät nach Anspruch 9 oder 10, **dadurch gekennzeichnet, dass** jeweils für eine Kombination aus den Elektrodenpolen, und/oder dem elektrischen Pol des Gerätegehäuses einzeln Impedanzwerte ausgewertet werden.

12. Medizinisches Gerät nach mindestens einem der Ansprüche 6 bis 11, **dadurch gekennzeichnet, dass** die Mess- und Steuereinheit ausgelegt ist, für eine Einzelmessung bei Überschreitung eines Schwellwertes durch die Impedanzwerte oder durch den berechneten Differenzwert einen Zählerwert zu inkrementieren, und bei Überschreitung eines Zählerlimits eine Speicherung der gemessenen Impedanzwerte auszulösen.

13. Medizinisches Gerät nach Anspruch 12, **dadurch gekennzeichnet, dass** die Mess- und Steuereinheit ausgelegt ist, eine Speicherung von Impedanzwerten einer Einzelmessung aus einer Reihe von Einzelmessungen zu veranlassen, die aus der Reihe von Einzelmessungen die Einzelmessung mit dem höchsten Zählerwert darstellt, oder
die die chronologisch erste Einzelmessung aus der Reihe von Einzelmessungen darstellt, für die das Zählerlimit überschritten wurde.

14. Medizinisches Gerät nach mindestens einem der Ansprüche 12 oder 13, **dadurch gekennzeichnet, dass** bei der Speicherung die Impedanzwerte innerhalb eines Unterzeitfensters gespeichert werden, wobei das Unterzeitfenster mit dem Zeitpunkt der Überschreitung des Zählerlimits verbunden ist.

15. Methode zur Auswertung von Impedanzwerten hinsichtlich eines Fehlers in einer Elektrodenleitung, umfassend die Schritte
• Messungen einer Impedanz über den Elektrodenpol einer Elektrodenleitung,
• Auswertung gemessener Impedanzwerte hinsichtlich eines Fehlers in der Elektrodenleitung,
**dadurch gekennzeichnet, dass** die Messungen der Impedanz zumindest eine Einzelmessung aufweist, und eine Einzelmessung über ein festgelegtes Zeitfenster stattfindet.
